# FASCICULE DE BREVET EUROPEEN

(11) **EP 1 684 625 B1**
(45) Date de publication et mention de la délivrance du brevet: **04.05.2011**
(21) Numéro de dépôt: 04805446.4
(22) Date de dépôt: 12.11.2004
(51) Int. Cl.: A61N 5/06, A61B 18/20, H05B 41/19

(54) **APPAREIL D'EPILATION ET PROCEDE DE MISE EN OEUVRE D'UN TEL APPAREIL**
HAARENTFERNUNGSVORRICHTUNG UND VERFAHREN ZUR ANWENDUNG EINER SOLCHEN VORRICHTUNG
HAIR-REMOVAL DEVICE AND METHOD OF USING ONE SUCH DEVICE

(30) Priorité: 13.11.2003 FR 0313263
(43) Date de publication de la demande: 02.08.2006
(73) Titulaire: Brottier, Yves Vincent, 92210 Saint Cloud (FR)
(72) Inventeur: BROTTIER, Yves, Vincent, 92210 Saint Cloud (FR); HUGUENY, Alain, Marie, F-69003 Lyon (FR)
(74) Mandataire: Vougny, Christophe
(86) Numéro de dépôt international: PCT/FR2004/002906
(87) Numéro de publication internationale: WO 2005/048808

(56) Documents cités:
- EP-A- 0 885 629
- FR-A- 2 838 042
- US-A- 4 550 275

## Description

La présente invention concerne les appareils d'épilation pour applications cutanées et locales, adaptés pour émettre au moins une impulsion lumineuse générée par une impulsion de courant électrique traversant une lampe flash en y formant un arc électrique, ce courant provenant de la décharge d'au moins un condensateur et étant commandé par un interrupteur électronique tout ou rien à commutation rapide, ces trois éléments étant disposés en une boucle principale. Par impulsion lumineuse on entend une impulsion de rayonnement visible et/ou infrarouge V/IR, généralement générée par une lampe flash au xénon.

Tous ces appareils produisent des impulsions dont la durée est comprise entre 5 et 50 ms (millisecondes), ce qui est exceptionnellement long pour un flash, et présente, pour cette raison, des problèmes spécifiques de régulation.

On sait par expérience que la réponse cutanée est la meilleure si les impulsions lumineuses V/IR présentent une forme assez carrée, avec une puissance instantanée assez s table pendant toute leur durée. Parmi les appareils existants et se rapprochant bien de ces caractéristiques, on peut citer les appareils à décharges trapézoïdales qui comportent en boucle : un condensateur, un interrupteur et une lampe flash. Document FR 2 838 042 décrit un appareil selon le préambule de la revendication 1.

Les deux inconvénients majeurs des appareils à décharges trapézoïdales sont la forte sollicitation de la lampe flash et le surdimensionnement du condensateur. Face à la forte sollicitation de la lampe, soit celle-ci est de fabrication ordinaire, bon marché en Borosilicate -aussi appelé couramment PYREX - elle s'use rapidement et doit être remplacée souvent, soit elle est fabriquée en Quartz pour offrir une meilleure durée de vie, mais à un prix beaucoup plus élevé. Quant au condensateur, son surdimensionnement est nécessaire dans les appareils actuels pour limiter la décroissance de puissance au cours de l'impulsion. Ce surdimensionnement présente l'inconvénient d'augmenter sensiblement le prix de fabrication des appareils, ainsi que leur volume et leur masse.

La présente invention vise à réduire la sollicitation de la lampe flash, et à améliorer sensiblement le taux d'utilisation du condensateur, dans le respect d'une puissance instantanée assez stable pendant l'impulsion lumineuse V/IR.

A cet effet, la présente invention a pour objet un appareil d'épilation du type précité, caractérisé en ce que pendant chaque décharge du condensateur, le courant traversant la lampe génère un signal de mesure représentatif dudit courant traversant la lampe, et en ce que ledit signal de mesure est comparé à une valeur de référence par un dispositif de comparaison à hystérésis pour commander l'état ouvert ou fermé dudit interrupteur et réguler ledit courant traversant la lampe par son découpage haute fréquence autour d'une intensité déterminée durant sensiblement toute la durée de ladite impulsion de courant.

Ainsi, une régulation active de l'intensité est effectuée au cours du bref moment pendant lequel dure l'impulsion de courant, ce qui permet de délivrer une impulsion lumineuse de puissance sensiblement constante et adaptée pour obtenir une bonne réponse cutanée. Contrairement aux dispositifs de l'art antérieur, il n'est pas nécessaire de surdimensionner le condensateur afin que la tension à ses bornes varie peu. De plus, cette régulation limite l'intensité maximum du courant traversant la lampe, et par conséquent le diamètre de l'arc électrique, ce qui préserve la lampe.

Dans des formes de réalisation préférées de l'invention, on a recours, en outre, à l'une et/ou à l'autre des dispositions suivantes :
- le signal de mesure est une tension de mesure proportionnelle au courant traversant la lampe, qui est fournie par un transducteur courant-tension, ledit transducteur courant-tension étant de préférence une résistance montée en série dans la boucle principale, ainsi le signal de mesure est fourni de manière directe et simple ;
- une diode de roue libre est incorporée dans une boucle additionnelle comportant, en commun avec la boucle principale, la lampe flash et le transducteur courant-tension, ce qui évite l'apparition de surtension pouvant endommager l'interrupteur électronique ;
- la boucle principale comporte en outre une inductance, atténuant la rapidité des variations de courant et facilitant la régulation de celui-ci ;
- le dispositif de comparaison à hystérésis et la valeur de référence sont choisis de manière à réguler le courant traversant la lampe à une valeur sensiblement inférieure à la valeur du courant pour laquelle l'expansion de l'arc électrique atteint les parois intérieures de la lampe, pour préserver la durée de vie de celle-ci ;
- ledit au moins un condensateur est choisi de manière à ce que le rapport entre la tension initiale à ses bornes avant décharge et la tension finale à la fin de l'impulsion de courant, soit compris entre 2 et 6, et de préférence de l'ordre de 4, de manière à optimiser le taux d'utilisation du condensateur et par conséquent à limiter sa capacité ;
- ledit au moins un condensateur présente une capacité au plus égale à 13 000 microfarads et une tension nominale au plus égale à 400 Volts, ledit au moins condensateur étant de préférence un condensateur électrolytique ;
- la capacité du condensateur est choisie de manière à ce que la valeur moyenne de l'intensité du courant traversant la lampe mesurée sur une période de une milliseconde à la fin de l'impulsion de courant, soit comprise entre 90% et 100% de la valeur moyenne de l'intensité mesurée sur une même période au début de l'impulsion de courant, de manière à obtenir une épilation efficace et sans douleur ;
- l'interrupteur électronique est choisi de manière à présenter un temps de commutation nettement inférieur à une microseconde, ledit commutateur étant de préférence un IGBT ;
- un module de commande électronique est adapté pour fournir la valeur de référence au dispositif de comparaison à hystérésis, et adapté pour commander la délivrance d'une impulsion de courant dans la boucle principale pendant une durée d'impulsion déterminée, ladite durée d'impulsion étant comprise entre 20ms et 45ms, et de préférence égale à environ 35ms pour obtenir une bon compromis entre l'épilation et la préservation des tissus cutanés ;
- le module de commande est adapté pour fournir à l'interrupteur à commutation rapide, une temporisation de durée déterminée à chaque ouverture de cet interrupteur, pendant laquelle il lui est interdit de basculer à nouveau à l'état fermé, le durée déterminée étant significativement plus longue que le temps de commutation de l'interrupteur mais inférieure au temps nécessaire à la lampe pour s'éteindre, et éviter ainsi un échauffement excessif de l'interrupteur ;
- le module de commande est adapté pour ne pas commander l'émission d'une nouvelle impulsion de courant avant qu'un temps déterminé, dit de repos, ne se soit écoulé depuis la précédente impulsion de courant, le temps de repos étant compris entre une seconde et dix secondes, et de préférence environ égal à sept secondes, ce qui a une influence favorable sur le dimensionnement de l'appareil et de son alimentation électrique ;
- ledit au moins un condensateur, l'interrupteur électronique et le dispositif de comparaison à hystérésis, sont logés dans un même boîtier, ledit boîtier présentant un volume inférieur à cinq litres ; et
- le poids de l'ensemble de l'appareil est au plus égal à deux kilos, de manière à être aisément transportable.

La présente invention a également pour objet un procédé de mise en oeuvre d'un appareil d'épilation tel que défini précédemment, caractérisé en ce qu'il comprend, au cours de la génération de l'impulsion de courant électrique, les étapes de :
- génération d'un signal de mesure représentatif du courant traversant la lampe ;
- comparaison avec hystérésis entre le signal de mesure et une valeur de référence ;
- commande de la fermeture de l'interrupteur électronique si la comparaison entre le signal de mesure et la valeur de référence indique que le courant traversant la lampe est inférieur à une intensité déterminée ; ou
- commande de l'ouverture de l'interrupteur électronique si la comparaison entre le signal de mesure et la valeur de référence indique que le courant traversant la lampe est supérieur à l'intensité déterminée, de manière à réguler ledit courant traversant la lampe par découpage de celui-ci à haute fréquence autour de l'intensité déterminée.

Dans un mode de mise en oeuvre préférée du procédé il est prévu, à chaque ouverture de l'interrupteur, une étape de temporisation de durée déterminée, pendant laquelle il est interdit à l'interrupteur de basculer à nouveau à l'état fermé, le temps déterminé étant significativement plus long que le temps de commutation de l'interrupteur, mais inférieur au temps nécessaire à la lampe pour s'éteindre.

En plus de la description qui précède, la présente invention sera mieux comprise à l'aide de la description qui va suivre, donnée à titre purement illustratif et non limitatif, en référence aux dessins dans lesquels :
- la figure 1 représente un schéma de base d'un appareil à impulsions trapézoïdales de l'art antérieur,
- la figure 2 montre un diagramme tension /temps, soit u/t, de la tension aux bornes du condensateur d'un appareil selon la figure 1 pendant une impulsion de type trapézoïdale,
- la figure 3 montre un diagramme puissance instantanée/temps, soit Pimp/t, de la lampe flash d'un appareil selon la figure 1 pendant une impulsion de type trapézoïdal,
- la figure 4 représente un schéma d'un mode de réalisation préféré d'un appareil selon la présente invention,
- la figure 5 montre un diagramme tension/temps, soit u/t, de la tension aux bornes du condensateur de la figure 4 pendant une impulsion,
- la figure 6 montre un diagramme puissance instantanée/temps, soit Pimp/t, de la lampe flash d'un appareil selon la figure 4 pendant une impulsion,
- la figure 7 est une vue en perspective d'un appareil à épiler selon l'invention.

Dans ce qui suit les termes et abréviations utilisés ont les significations suivantes :
- condensateur "CE", signifie un ou plusieurs condensateurs électrolytiques, arrangés en série ou parallèle ou série-parallèle et destiné(s) à stocker de l'énergie électrique en vue de sa restitution, totale ou partielle, à la lampe flash,
- par impulsion "V/IR" on désigne l'impulsion visible et/ou infrarouge destinée à produire sur la peau l'effet d'épilation,
- par interrupteur "SW" on désigne un interrupteur électronique capable de commutations rapides,
- lampe "LF" signifie la ou les lampes flash, arrangée(s) en série ou parallèle ou série-parallèle,
- Ui représente la tension aux bornes de CE au début d'une impulsion (Ui pour tension initiale),
- Uf représente la tension aux bornes de CE à la fin d'une impulsion (Uf pour tension finale).

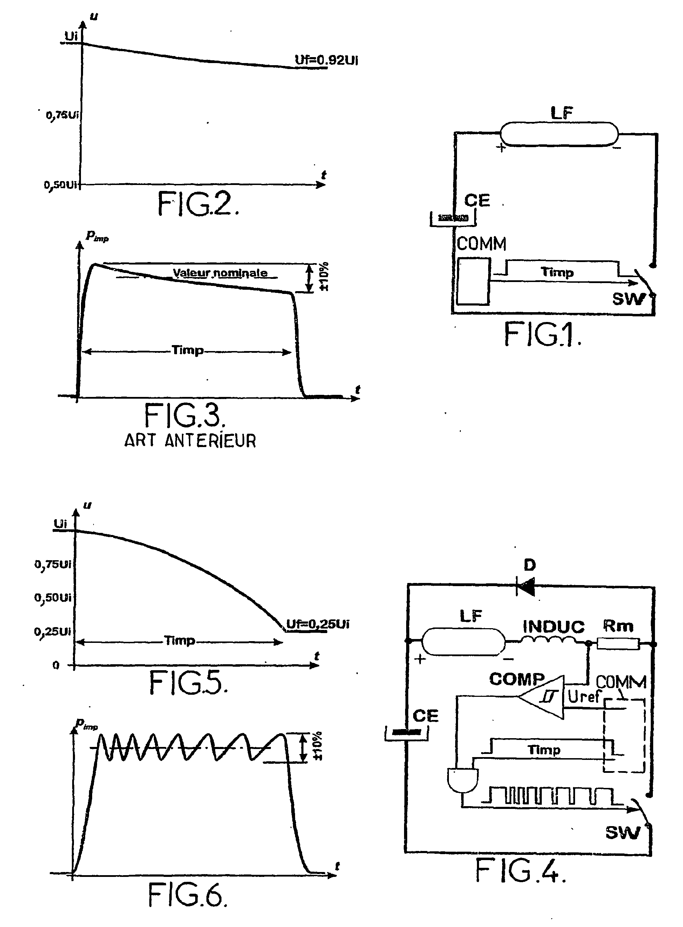

La figure 1 montre une lampe LF, un condensateur CE et un interrupteur SW disposés en boucle. L'interrupteur SW est commandé dans l'état ouvert ou fermé par une impulsion rectangulaire provenant d'un module de commande électronique COMM classique.

Suivant les fig. 2 et 3, le fonctionnement de cet appareil de l'art antérieur se décompose en trois phases :
1°) Allumage de la lampe alors que le condensateur CE est chargé à une tension Ui. Pendant cette phase d'allumage, l'arc électrique d'abord capillaire, et consommant un courant faible, augmente naturellement de diamètre jusqu'à entrer en contact avec la paroi interne de la lampe. Cette augmentation de diamètre, ou expansion, génère une réduction correspondante de l'impédance de la lampe et donc une augmentation du courant. L'ordre de grandeur de temps de ce phénomène se situe vers 100µs.
2°) Maintien en l'état allumé de la lampe, grâce au maintien en l'état fermé de l'interrupteur SW. Pendant toute cette phase la tendance naturelle à l'expansion de l'arc est interdite car il bute déjà sur la paroi interne de la lampe ; on dit que l'arc est confiné. De ce fait, il n'y a plus de croissance du courant, et même une décroissance en raison de la baisse de tension aux bornes du condensateur CE qui est en cours de décharge. Le contact physique entre l'arc électrique et la paroi de la lampe est un élément majeur de vieillissement de celle-ci. Mais ce contact est nécessaire pour imposer à l'arc électrique de conserver, pendant toute la durée de l'impulsion souhaitée, une section faible, donc une impédance relativement élevée et une puissance instantanée dans les limites souhaitées malgré la tension élevée nécessaire au fonctionnement de toute lampe flash. L'ordre de grandeur de puissance développée dans la lampe pendant cette phase est de 10kW, pour une durée de quelques dizaines de millisecondes.
3°) Interruption du fonctionnement de la lampe par ouverture de l'interrupteur SW au moment où l'impulsion lumineuse V/IR a duré le temps souhaité. A ce moment la tension aux bornes du condensateur CE est Uf. La tension Uf est inférieure à Ui en raison de l'énergie que le condensateur CE a restituée à la lampe. Rapidement après l'ouverture de l'interrupteur SW, l'arc électrique qui n'est plus alimenté, se contracte sur lui-même, son diamètre diminue et il fini par s'éteindre. Ce phénomène dont l'ordre de grandeur de temps se situe vers 100µs, se trouve allongé par l'inductance du câblage, ou par une inductance éventuellement intégrée dans le circuit.

Les paramètres théoriques et empiriques qui régissent la puissance développée dans une lampe flash fonctionnant en mode confiné sont tels que l'appareil de l'art antérieur est équipé de lampes présentant un rapport LA/DA (avec LA pour longueur de l'arc, et DA pour diamètre de l'arc) grand, généralement compris entre 10 et 50.

Il faut aussi savoir que la puissance instantanée d'émission d'une lampe flash contenant un arc confiné, est proportionnelle à la tension U à ses bornes puissance 2,5 ; U étant la tension aux bornes de la lampe (certaines formules donnent même U puissance 3, mais dans la suite de ce document, nous nous basons sur 2,5 pour les valeurs chiffrées mentionnées à titre d'exemples). En raison de cette relation entre la puissance et la tension, et pour assurer une différence raisonnablement faible entre la puissance instantanée au début de l'impulsion lumineuse V/IR et la puissance instantanée en fin d'impulsion, la différence entre Ui et Uf, doit être très faible, et seule une faible partie de l'énergie initialement stockée dans le condensateur est utilisée pour l'impulsion afin de limiter la baisse de tension à ses bornes. Le condensateur se trouve sous-utilisé et doit donc être de grande valeur capacitive. Par exemple, pour que la puissance instantanée reste dans la marge de + ou - 10% de sa valeur nominale, il faut avoir Uf = 0,92 x Ui, et dans ce cas, seulement 16% de l'énergie initialement stockée dans le condensateur CE est utilisée. Ainsi, pour être capable de délivrer 100 J, dans les conditions précédemment décrites, le condensateur CE doit stocker 625 J avant le début d'une impulsion. Ceci représente un surdimensionnement d'un facteur 6.

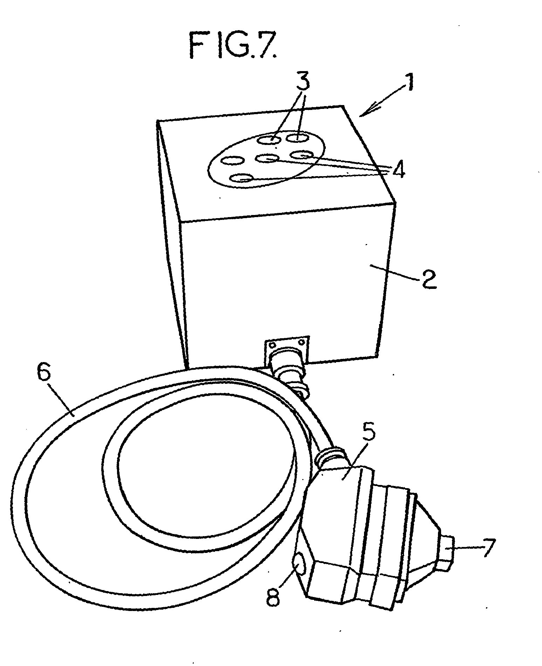

Comme représenté aux fig. 4, 5 et 6, la présente invention consiste à introduire une régulation de courant dans l'appareil d'épilation 1 représenté à la figure 7, notamment de manière à ce que l'arc électrique conserve une section inférieure à la section interne de la lampe tout en délivrant la puissance nécessaire.

De cette façon, l'arc électrique n'exerçant pas de pression intense sur la paroi interne de la lampe flash LF, elle vieillit moins vite, et d'autre part, avec la régulation du courant, la lampe peut rester à une puissance sensiblement constante pendant toute la durée de l'impulsion lumineuse V/IR, même si la différence de tension entre le début Ui et la fin Uf de l'impulsion est grande. Cette différence mène à un taux d'utilisation élevé du condensateur CE qui n'a ainsi plus besoin d'être surdimensionné.

La régulation par le courant est choisie car, pour une lampe flash donnée, avec un arc confiné ou non confiné, à courant constant correspond une puissance très stable ; alors qu'à tension constante correspond une puissance plus ou moins variable.

Comme représenté à la figure 7, l'appareil 1 comprend un boîtier 2 muni de boutons marche/arrêt 3 et de boutons de réglages 4 sur sa face supérieure, une pièce à main 5 et un cordon électrique 6 s'étendant du boîtier à la pièce à main. La pièce à main 5 contient une lampe flash au xénon et présente un guide de sortie de lumière 7, ainsi qu'un bouton 8 de déclenchement de l'impulsion lumineuse.

Selon la figure 4, un mode de réalisation préféré du dispositif selon la présente l'invention est agencé de la manière suivante :
La boucle principale du circuit de puissance du dispositif, désignée par boucle CE-SW-Rm-INDUC-LF, comporte le condensateur CE, un interrupteur SW, une résistance de mesure Rm, une inductance INDUC et la lampe flash LF.

Le condensateur CE formé d'un ou plusieurs condensateurs électrolytiques disposés en parallèle, en série ou en série-parallèle, dont la capacité nominale totale est de préférence inférieure ou égale à environ 13 000 microfarads dans le cas où l'on souhaite réaliser un appareil aisément transportable. Mais la capacité nominale peut être bien plus élevée pour un appareil de type professionnel. De même, la tension nominale d'utilisation du ou des condensateurs est au plus de 400 volts pour limiter leur coût. De plus, des tensions plus élevées augmenteraient l'isolation électrique nécessaire, ce qui irait à l'encontre de la réduction de volume de l'appareil. Les valeurs indiquées ci-dessus pour des modes particuliers de réalisation représentent des maximums, il est possible de diminuer la capacité ou la tension du condensateur lorsque respectivement la tension ou la capacité nominale s'approche de ces maximums.

L'interrupteur SW est un interrupteur électronique à commutation rapide, de l'ordre de 50 à 300 nanosecondes, et de préférence nettement inférieur à une microseconde. Pour cela, on choisit un interrupteur de type transistor bipolaire à porte isolée IGBT (Insulated Gate Bipolar Transistor), et éventuellement plusieurs interrupteurs de ce type peuvent être montés en parallèle.

La résistance de mesure Rm est une résistance de faible valeur ohmique pouvant supporter des intensités importantes. Elle permet de fournir de manière immédiate un signal de mesure sous forme d'une tension URm, qui est directement proportionnelle à l'intensité la traversant. Mais la présente invention n'exclut pas l'utilisation de tout autre transducteur courant-tension analogique ou digital, ni même de tout dispositif fournissant directement une mesure de l'intensité traversant la lampe flash LF.

L'inductance INDUC n'est pas indispensable, mais en raison du découpage haute fréquence qui prend place dans le circuit pour contrôler le transfert d'énergie, on préfère incorporer une inductance de lissage INDUC dans la boucle CE-SW-Rm-LF, par exemple entre la résistance Rm et la lampe LF.

Une diode de roue libre D est disposée dans une boucle additionnelle Rm-INDUC-LF-D. La diode D remplit deux rôles :
- assurer le transfert d'énergie depuis l'inductance INDUC vers la lampe LF lorsque l'interrupteur SW est ouvert et que de l'énergie électrique est emmagasinée dans l'inductance INDUC,
- limiter les pointes de tension aux bornes de l'interrupteur SW lors de son ouverture, pointes de tension qui sont générées par l'inductance INDUC, ainsi que par les inductances parasites du câblage qui sont relativement importantes du fait du courant de l'ordre de 250 ampères qui circule dans la boucle principale.

Les éléments de régulation comprennent au moins un comparateur de tension à hystérésis COMP et une porte logique.

Le comparateur COMP reçoit en entrée une valeur de tension de référence Uref fournie par un module de commande électronique COMM, et la tension de mesure URm générée aux bornes de la résistance Rm. Le comparateur fonctionne avec hystérésis, c'est-à-dire que lorsque la tension croît il fournit un signal indiquant un dépassement de la tension de référence Uref quand la tension mesurée URm dépasse la valeur de référence plus un écart déterminé, et inversement lorsque la tension décroît, le signal bascule quand la tension mesurée devient inférieure à la tension de référence moins un écart déterminé.

La tension de référence Uref est ajustée en fonction du courant moyen auquel on souhaite faire travailler la lampe LF, notamment en fonction du type de peau, par exemple en actionnant l'un des boutons de réglage 4 reliés au module de commande COMM qui contient plusieurs tensions de références mémorisées.

La porte logique présente une entrée reliée au comparateur COMP et une entrée reliée au module de commande COMM de manière à ne transmettre une commande de fermeture de l'interrupteur SW que pendant la durée d'impulsion. A cet effet, le module de commande COMM est adapté pour délivrer un signal Timp pendant une durée déterminée correspondant à la durée de l'impulsion de courant, suite à l'actionnement du bouton de déclenchement 8.

La durée d'impulsion pourrait être réglable, toutefois les tests montrent qu'une durée constante comprise entre 20 et 45 ms, et de préférence environ égale à 35 ms, permet une épilation efficace de tous les types de peau.

Bien que la présente invention n'exclue pas l'envoi par le module de commande d'une série de signaux d'impulsion de manière à produire une série d'impulsions lumineuses distinctes, dans un mode de réalisation particulier on préfère interdire l'émission d'une nouvelle impulsion lumineuse avant qu'un temps déterminé, dit de repos, très nettement supérieur à la durée d'une impulsion ne se soit écoulé. Le temps de repos programmé dans le module de commande COMM, est de l'ordre de quelques secondes, entre une et dix secondes, et de préférence d'environ sept secondes. Ce temps de repos relativement long permet de s'assurer que le condensateur a atteint sa charge initiale, c'est-à-dire la tension Ui, sans qu'il soit nécessaire de prévoir une alimentation électrique de recharge du condensateur de forte puissance. De plus, ce temps de repos peut être mis a profit pour refroidir la lampe flash LF et l'interrupteur SW par convection naturelle, ou éventuellement par convection forcée mais de faible débit, ce qui est favorable à la réduction du poids et de l'encombrement de l'appareil.

Comme l'indiquent les fig. 5 et 6, préalablement à la génération d'une impulsion lumineuse V/IR, le condensateur CE est chargé à la tension initiale Ui par un dispositif d'alimentation électrique non représenté. Le dispositif d'alimentation électrique qui peut être de tout type connu, génère une tension continue qui est fournie aux bornes du condensateur CE.

Lors de la conception d'un dispositif selon la présente invention, il faut d'abord connaître la puissance de rayonnement Pimp souhaitée pendant l'impulsion, la durée de l'impulsion Timp et la longueur de la lampe Larc. Avec ces paramètres, on peut, soit par calcul grâce aux formules fournies par les fabricants de lampes, soit expérimentalement, trouver la différence de potentiel à appliquer aux extrémités d'une lampe flash expérimentale de diamètre important et de longueur Larc, dans laquelle un arc électrique stabilisé et non confiné génère un rayonnement de puissance Pimp. La valeur trouvée est appelée Ucritic, elle correspond à une valeur de courant Icritic. Une expérience ou un calcul du même type renseignent sur le diamètre de l'arc électrique ; ce diamètre est nommé Dcritic. Au-delà de l'étape expérimentale, pour les appareils fonctionnels, on retient Dlamp = Dcritic x 1,4 où Dlamp est le diamètre intérieur de la lampe. Pour la tension finale Uf, on retient une valeur voisine de Ucritic x 1,25, et pour la tension initiale Ui, une valeur voisine de Uf x 4. Les coefficients qui viennent d'être utilisés (1,4 1,25 et 4) peuvent être modulés dans une assez large mesure, mais chacun d'eux est supérieur à 1.

Pour la boucle principale du circuit de puissance CE-SW-Rm-INDUC-LF, on préfère, mais de façon non limitative, une disposition des éléments dans l'ordre suivant : le pôle négatif du condensateur CE, l'interrupteur SW, la résistance de mesure Rm (ou autre transducteur courant-tension), l'inductance INDUC, la lampe LF (pôle négatif coté inductance) et le pôle positif du condensateur CE. La boucle additionnelle est formée de la résistance de mesure (ou autre transducteur courant-tension) Rm, l'inductance INDUC, la lampe LF et la diode D. La diode D ayant, dans ce cas, sa cathode connectée aux pôles positifs de la lampe LF et du condensateur CE.

La valeur inductive de INDUC peut être telle que lorsqu'elle est parcourue par un courant de valeur Icritic elle emmagasine une énergie électrique comprise entre le 1/100 et 1/1000 de l'énergie électrique transférée à la lampe LF lors de chaque impulsion lumineuse V/IR.

Pour démarrer une impulsion, la lampe flash LF est rendue conductrice, par exemple au moyen d'une impulsion très haute tension selon une méthode classique et connue pour les lampes flash, et assurer la fermeture de l'interrupteur SW. A ce moment le courant, d'abord nul, croît dans la boucle CE-SW-Rm-INDUC-LF car l'arc électrique augmente de section et l'inductance INDUC ralentit l'évolution du courant. Ce courant crée une différence de tension URm aux bornes de Rm. Lorsque URm a dépassé Uref, la sortie du comparateur COMP change d'état. Ce changement d'état commande l'ouverture de l'interrupteur SW. Une fois l'interrupteur SW ouvert, le courant dans la boucle Rm-INDUC-LF-D, ainsi que la tension URm, décroît jusqu'à un seuil qui génère à nouveau le basculement de la sortie du comparateur COMP. Ce basculement commande la fermeture de l'interrupteur SW, si le temps de l'impulsion Timp n'est pas écoulé. Pour une impulsion complète, ces cycles se reproduisent autant de fois que nécessaire, par exemple de l'ordre d'une centaine de fois pour une durée d'impulsion de 35 ms. On notera que l'intensité du courant circulant dans la lampe flash ne chute pas à une valeur nulle immédiatement après l'ouverture de l'interrupteur, même en l'absence de l'inductance INDUC. En effet, étant donné les intensités et tensions élevées dans les boucles principale et secondaire, ainsi que les phénomènes d'ionisation dans la lampe, le câblage et la lampe emmagasinent une certaine énergie électrique qui est restituée lors de l'ouverture de l'interrupteur SW. Par contre, le courant traversant le condensateur CE qui est en dehors de la boucle secondaire, s'annule très rapidement. On peut donc constater aux bornes du condensateur l'émission d'impulsions élémentaires de courant rapprochées.

A l'intérieur de chaque cycle le courant qui traverse Rm (et donc celui qui traverse la lampe LF) est régulé autour de la valeur de courant Icritic, avec une ondulation dont l'amplitude dépend de l'hystérésis du comparateur COMP. Cette régulation est réalisée sur sensiblement toute la durée de l'impulsion, c'est-à-dire sur la durée de l'impulsion moins la durée les phases d'allumages de la lampe et d'extinction du courant qui sont très brèves, de l'ordre de 100 microsecondes, en comparaison de la durée de l'impulsion, de l'ordre de 40 millisecondes.

L'amplitude des ondulations de courant dans la lampe LF, et par conséquent de la puissance schématiquement représentée à la figure 6, est de + ou - 10%, mais l'on peut autoriser des amplitudes supérieures à + ou - 30% étant donné leur durée très brève et la succession rapide de pics et de creux. Par contre, il est important que sur une durée significative par rapport à l'inertie thermique des tissus cutanés, notamment sur une durée de 1 ms ou la durée d'un cycle ouverture-fermeture de l'interrupteur SW, la puissance moyenne de l'impulsion Pimp soit proche de la puissance correspondant à l'intensité de régulation Icritic, et de préférence égale à celle-ci à + ou - 10 %. D'autre part, une meilleure réponse cutanée est obtenue si l'intensité moyenne du courant traversant la lampe ne décroît que faiblement, de moins de 10%, entre le début et la fin de l'impulsion.

Les valeurs de puissance instantanée mises en jeu étant très élevées, il faut porter une attention particulière à l'interrupteur SW dont il a été mentionné que c'est un commutateur tout ou rien rapide. Le mot rapide se rapporte à ses changements d'état, qui durent quelques centaines de nanosecondes, mais pendant chaque commutation il est le siège d'une dissipation thermique instantanée très importante. Cette dissipation thermique moyenne reste acceptable s'il n'y a pas de successions trop rapides de commutations. Pour garantir cette condition on peut augmenter l'hystérésis sur la régulation du courant à travers la lampe LF, ou incorporer dans la commande de l'interrupteur SW une temporisation T_{interdit} (non représentée) de quelques dizaines de microsecondes qui impose le maintien de l'interrupteur SW à l'état ouvert après chaque passage de l'état fermé à l'état ouvert. De cette façon, l'interrupteur SW ne peut pas être sollicité pour des cycles très rapides fermeture-ouverture-fermeture, et lorsque la temporisation T_{interdit} intervient, elle contribue à réduire la puissance de transfert d'énergie à la lampe, ce qui peut altérer la qualité du résultat fonctionnel, mais place l'interrupteur SW dans une zone de travail mieux sécurisée. Lorsque la temporisation T_{interdit} intervient, le courant traversant la lampe LF décroît sous la valeur correspondant à la commande de fermeture de : l'interrupteur générée par le comparateur à hystérésis, et peut devenir nul. Mais dans ce cas l'annulation du courant dans la lampe n'intervient que pendant une durée inférieure à la durée de temporisation qui est elle-même inférieure à la durée d'extinction de la lampe. La puissance lumineuse émise par la lampe ne s'annule donc pas et augmente dès la fin de la temporisation car la lampe flash demeure passante. L'impulsion lumineuse n'est pas interrompue et cette baisse ou annulation du courant dans la lampe n'a pas d'effet néfaste sur l'épilation étant donné sa brièveté.

La partie des circuits qui génère la temporisation T_{interdit'} si cette partie optionnelle existe, est placée dans la chaîne des signaux près de l'interrupteur SW. De cette façon, en présence d'un éventuel signal parasite perturbant les circuits, l'interrupteur SW a les meilleures chances d'être protégé contre une sollicitation accidentelle à fréquence trop élevée. En l'absence de perturbation électromagnétique exceptionnelle, la sécurité apportée par T_{interdit} n'intervient pas. A titre d'exemple, cette temporisation T_{interdit} peut être réalisée par un signal émis par le module de commande COMM vers une porte logique supplémentaire recevant également le signal de la porte logique représentée à la figure 4.

Avec un tel procédé de régulation, il est théoriquement possible de choisir une valeur de Ui très élevée par rapport à Uf, par exemple Ui = 10 x Uf. Dans ce cas, le condensateur CE serait utilisé à 99%, mais l'interrupteur SW devrait supporter une tension élevée et son prix augmenterait exagérément pour certains modes de réalisation. Un bon compromis entre les aspects techniques et financiers consiste à choisir le rapport Ui/Uf entre 3 et 5, voir 6. A la fig.5, avec Ui = 4 x Uf, après une impulsion il ne reste que 1/16ième de l'énergie initialement stockée dans CE. Le taux d'utilisation de CE est de 15/16, soit de 93,7% contre 16% dans l'exemple cité relativement à l'art antérieur à la fig. 2. Avec un rapport Ui/Uf de 2, on obtient déjà un taux d'utilisation du condensateur CE supérieur à 60%.

La figure 6 représente à titre indicatif, un résultat qui peut être obtenu avec la puissance instantanée qui reste dans la marge de + ou - 10% de sa valeur nominale pendant une décharge lumineuse V/IR, alors que le taux d'utilisation de CE est de 93,7% pendant la même décharge.

L'invention permet de réaliser des appareils plus petits, de fabrication plus économique, et consommant des lampes moins onéreuses que les appareils de l'art antérieur. En choisissant un ou des condensateurs appropriés et en optimisant leur utilisation grâce au différentes dispositions de la présente invention, il est possible de réaliser un appareil de série d'un poids inférieur à 2 Kg. En limitant la tension d'utilisation du ou des condensateurs, et grâce à la régulation par découpage haute fréquence, le boîtier 2 qui contient l'alimentation électrique permettant de recharger le condensateur, le condensateur, l'interrupteur, la résistance, l'inductance et le module de commande, présente des dimensions réduites de sorte que le volume du boîtier est inférieur à 5 litres.

## Revendications

1. Appareil d'épilation (1) pour applications cutanées et locales, adapté pour émettre au moins une impulsion lumineuse générée par une impulsion de courant électrique traversant une lampe flash (LF) en y formant un arc électrique, ce courant provenant de la décharge d'au moins un condensateur (CE) et étant commandé par un interrupteur électronique (SW) tout ou rien à commutation rapide, ces trois éléments étant disposés en une boucle principale (CE-SW-LF), **caractérisé en ce que** pendant chaque décharge du condensateur, le courant traversant la lampe (LF) génère un signal de mesure (URm) représentatif dudit courant traversant la lampe, et **en ce que** ledit signal de mesure (URm) est comparé à une valeur de référence (Uref) par un dispositif de comparaison à hystérésis (COMP) pour commander l'état ouvert ou fermé dudit interrupteur (SW) et réguler ledit courant traversant la lampe (LF) par son découpage haute fréquence autour d'une intensité déterminée (Icritic) durant sensiblement toute la durée de ladite impulsion de courant.

2. Appareil selon la revendication 1, dans lequel le signal de mesure est une tension de mesure (URm) proportionnelle au courant traversant la lampe (LF), qui est fournie par un transducteur courant-tension, ledit transducteur courant-tension étant de préférence une résistance (Rm) montée en série dans la boucle principale (CE-SW-LF).

3. Appareil selon la revendication 2, dans lequel une diode de roue libre (D) est incorporée dans une boucle additionnelle (Rm-LF-D) comportant, en commun avec la boucle principale, la lampe flash (LF) et le transducteur courant-tension (Rm).

4. Appareil selon l'une quelconque des revendications 1 à 3, dans lequel la boucle principale (CE-SW-LF) comporte en outre une inductance (INDUC).

5. Appareil selon l'une quelconque des revendications précédentes, dans lequel le dispositif de comparaison à hystérésis (COMP) et la valeur de référence (Uref) sont choisis de manière à réguler le courant traversant la lampe (LF) à une valeur sensiblement inférieure à la valeur du courant pour laquelle l'expansion de l'arc électrique atteint les parois intérieures de la lampe (LF).

6. Appareil selon l'une quelconque des revendications précédentes, dans lequel ledit au moins un condensateur (CE) est choisi de manière à ce que le rapport entre la tension initiale à ses bornes (Ui) avant décharge et la tension finale (Uf) à la fin de l'impulsion de courant, soit compris entre 2 et 6, et de préférence de l'ordre de 4.

7. Appareil selon l'une quelconque des revendications précédentes, dans lequel ledit au moins un condensateur présente une capacité au plus égale à 13 000 microfarads et une tension nominale au plus égale à 400 Volts, ledit au moins un condensateur étant de préférence un condensateur électrolytique.

8. Appareil selon l'une quelconque des revendications précédentes, dans lequel la capacité du condensateur (CE) est choisie de manière à ce que la valeur moyenne de l'intensité du courant traversant la lampe (LF) mesurée sur une période de une milliseconde à la fin de l'impulsion de courant, soit comprise entre 90% et 100% de la valeur moyenne de l'intensité mesurée sur une même période au début de l'impulsion de courant.

9. Appareil selon l'une quelconque des revendications précédentes, dans lequel l'interrupteur électronique (SW) est choisi de manière à présenter un temps de commutation nettement inférieur à une microseconde, ledit commutateur étant de préférence un IGBT.

10. Appareil selon l'une quelconque des revendications précédentes, dans lequel un module de commande électronique (COMM) est adapté pour fournir la valeur de référence (Uref) au dispositif de comparaison à hystérésis (COMP), et adapté pour commander la délivrance d'une impulsion de courant dans la boucle principale pendant une durée d'impulsion déterminée, ladite durée d'impulsion étant comprise entre 20ms et 45ms, et de préférence égal à environ 35ms.

11. Appareil selon la revendication 10, dans lequel le module de commande (COMM) est adapté pour fournir à l'interrupteur à commutation rapide (SW), une temporisation (Tinterdit) de durée déterminée à chaque ouverture de cet interrupteur (SW), pendant laquelle il lui est interdit de basculer à nouveau à l'état fermé, le durée déterminée étant significativement plus longue que le temps de commutation de l'interrupteur (SW) mais inférieure au temps nécessaire à la lampe (LF) pour s'éteindre.

12. Appareil selon la revendication 10 ou 11, dans lequel le module de commande (COMM) est adapté pour ne pas commander l'émission d'une nouvelle impulsion de courant avant qu'un temps déterminé, dit de repos, ne se soit écoulé depuis la précédente impulsion de courant, le temps déterminé de repos étant compris entre une seconde et dix secondes, et de préférence environ égal à sept secondes.

13. Appareil selon l'une quelconque des revendications précédentes, dans lequel ledit au moins un condensateur (CE), l'interrupteur électronique (SW) et le dispositif de comparaison à hystérésis (COMP), sont logés dans un même boîtier, ledit boîtier présentant un volume inférieur à cinq litres.

14. Appareil selon l'une quelconque des revendications précédentes, dans lequel le poids de l'ensemble de l'appareil est au plus égal à deux kilos.

15. Procédé de mise en oeuvre d'un appareil d'épilation adapté pour émettre au moins une impulsion lumineuse générée par une impulsion de courant électrique traversant une lampe flash (LF) en y formant un arc électrique, dans lequel on fournit au moins un condensateur (CE) adapté pour délivrer le courant traversant la lampe flash, un interrupteur électronique (SW) tout ou rien à commutation rapide, le condensateur (CE), l'interrupteur électronique (SW) et la lampe flash (LF) formant une boucle principale (CE-SW-LF), **caractérisé en ce qu'**il comprend, au cours de la génération de l'impulsion de courant électrique, les étapes de :
- génération d'un signal de mesure (URm) représentatif du courant traversant la lampe (LF) ;
- comparaison avec hystérésis entre le signal de mesure (URm) et une valeur de référence (Uref);
- commande de la fermeture de l'interrupteur électronique (SW) si la comparaison entre le signal de mesure (URm) et la valeur de référence (Uref) indique que le courant traversant la lampe (LF) est inférieur à une intensité déterminée (Icritic) ; ou
- commande de l'ouverture de l'interrupteur électronique (SW) si la comparaison entre le signal de mesure (URm) et la valeur de référence (Uref) indique que le courant traversant la lampe (LF) est supérieur à l'intensité déterminée (Icritic), de manière à réguler ledit courant traversant la lampe (LF) par découpage de celui-ci à haute fréquence autour de l'intensité déterminée (I critic).

16. Procédé selon la revendication 15, dans lequel est prévu, à chaque ouverture de l'interrupteur (SW), une étape de temporisation (Tinterdit) de durée déterminée, pendant laquelle il est interdit à l'interrupteur (SW) de basculer à nouveau à l'état fermé, le temps déterminé étant significativement plus long que le temps de commutation de l'interrupteur (SW) mais inférieur au temps nécessaire à la lampe pour s'éteindre.

## Claims

1. A hair-removal device for local skin application, designed to emit at least one light pulse generated by an electric current pulse passing through a flashlamp (LF) forming an electric arc therein, this current coming from the discharge of at least one capacitor (CE) and being controlled by a fast-switching on/off electronic switch (SW), these three elements being placed in a main loop (CE-SW-LF), **characterized in that**, during each discharge of the capacitor, the current passing through the flashlamp (LF) generates a measurement signal (URₘ) representative of said current passing through the flashlamp, and **in that** said measurement signal (URₘ) is compared with a reference value (U_{ref}) by a hysteresis comparator (COMP) in order to set said switch (SW) in the open or closed state and to regulate said current passing through the flashlamp (LF) by its high-frequency cutoff around a defined current (I_{critic}) through substantially the entire duration of said current pulse.

2. The device as claimed in claim 1, wherein the measurement signal is a measurement voltage (URₘ) proportional to the current passing through the flashlamp (LF), which voltage is delivered by a current-voltage transducer, said current-voltage transducer preferably being a resistor (Rₘ) connected in series in the main loop (CE-SW-LF).

3. The device as claimed in claim 2, wherein a freewheeling diode (D) is incorporated into an additional loop (Rₘ-LF-D) comprising, in common with the main loop, the flashlamp (LF) and the current-voltage transducer (Rₘ).

4. The device as claimed in any one of claims 1 to 3, wherein the main loop (CE-SW-LF) further includes an inductor (IND).

5. The device as claimed in any one of the preceding claims, wherein the hysteresis comparator (COMP) and the reference value (U_{ref}) are chosen so as to regulate the current passing through the flashlamp (LF) to a value substantially below the value of the current for which the expansion of the electric arc reaches the inner walls of the flashlamp (LF).

6. The device as claimed in any one of the preceding claims, wherein said at least one capacitor (CE) is chosen so that the ratio of the initial voltage (Uᵢ) across its terminals before discharge to the final voltage (U_{f}) at the end of the current pulse is between 2 and 6, and preferably about 4.

7. The device as claimed in any one of the preceding claims, wherein said at least one capacitor has a capacitance at most equal to 13 000 microfarads and a nominal voltage at most equal to 400 volts, said at least one capacitor preferably being an electrolytic capacitor.

8. The device as claimed in any one of the preceding claims, wherein the capacitance of the capacitor (CE) is chosen so that the mean value of the current flowing through the flashlamp (LF), measured over a period of one millisecond at the end of the current pulse, is between 90% and 100% of the mean value of the current measured over the same period at the start of the current pulse.

9. The device as claimed in any one of the preceding claims, wherein the electronic switch (SW) is chosen so as to have a switching time considerably shorter than one microsecond, said switch preferably being an IGBT.

10. The device as claimed in any one of the preceding claims, wherein an electronic control module (COMM) is designed to deliver the reference value (U_{ref}) to the hysteresis comparator (COMP) and designed to deliver a current pulse in the main loop over a specified pulse duration, said pulse duration being between 20 ms and 45 ms, and preferably equal to about 35 ms.

11. The device as claimed in claim 10, wherein the control module (COMM) is designed to introduce, into the fast-switching switch (SW), a delay (T_{interdit}) of specified duration at each opening of this switch (SW), during which it is prevented from switching again to the closed state, the specified duration being significantly longer than the switching time of the switch (SW) but shorter than the time needed for the flashlamp (LF) to be turned off.

12. The device as claimed in claim 10 or 11, wherein the control module (COMM) is designed so as not to cause a new current pulse to be emitted before a specified time, called the rest time, has elapsed since the preceding current pulse, the specified rest time being between one second and ten seconds, and preferably about seven seconds.

13. The device as claimed in any one of the preceding claims, wherein said at least one capacitor (CE), the electronic switch (SW) and the hysteresis comparator (COMP) are housed in a same casing, said casing having a volume of less than five liters.

14. The device as claimed in any one of the preceding claims, wherein the weight of the overall device is at most equal to two kilograms.

15. A method of employing a hair-removal device designed to emit at least one light pulse generated by an electric current pulse passing through a flashlamp (LF) forming an electric arc therein, wherein at least one capacitor (CE), designed to deliver the current passing through the flashlamp, and a fast-switching on/off electronic switch (SW) are provided, the capacitor (CE), the electronic switch (SW) and the flashlamp (LF) forming a main loop (CE-SW-LF), **characterized in that** it comprises, while generating the electric current pulse, the steps of:
- generating a measurement signal (URₘ) representative of the current passing through the flashlamp (LF);
- comparing, with hysteresis, the measurement signal (URₘ) with a reference value (U_{ref}) ;
- closing the electronic switch (SW) if the comparison between the measurement signal (URₘ) and the reference value (U_{ref}) indicates that the current passing through the flashlamp (LF) is below a specified current (I_{critic}) ; or
- opening the electronic switch (SW) if the comparison between the measurement signal (URₘ) and the reference value (U_{ref}) indicates that the current passing through the flashlamp (LF) is above the specified current (I_{critic}), so as to regulate said current passing through the flashlamp (LF) by high-frequency cutoff thereof around the specified current (I_{critic}).

16. The method as claimed in claim 15, wherein, each time the switch (SW) is opened, a delay step (T_{interdit}) of specified duration is provided, during which the switch (SW) is prevented from switching again to the closed state, the specified time being significantly longer than the switching time of the switch (SW) but shorter than the time needed for the flashlamp to be turned off.

## Patentansprüche

1. Apparat zur Haarentfernung (1) für Haut- und lokale Anwendungen, der zur Aussendung zumindest eines Lichtpulses angepasst ist, welcher durch einen eine Blitzlichtlampe (LF) durchfließenden und darin einen Lichtbogen bildenden elektrischen Strompuls erzeugt wird, wobei dieser Strom aus der Entladung zumindest einen Kondensators (CE) stammt und durch einen schnellschaltenden elektronischen An/Aus-Schalter (SW) gesteuert wird, wobei diese drei Elemente in einer Hauptschleife (CE-SW-LF) angeordnet sind, **dadurch gekennzeichnet, dass** der die Lampe (LF) durchfließende Strom während jeder Entladung des Kondensators ein Messsignal (URm) erzeugt, das für den besagten die Lampe durchfließenden Strom repräsentativ ist, und dass das besagte Messsignal (URm) durch eine Hysterese-Vergleichsvorrichtung (COMP) mit einem Referenzwert (Uref) zur Steuerung des offenen oder geschlossenen Zustands des besagten Schalters (SW) und zur Kompensation des besagten die Lampe (LF) durchfließenden Stroms über sein hochfrequentes Schalten um eine bestimmte, im Wesentlichen die gesamte Dauer des besagten Strompulses dauernde Intensität (Icritic) verglichen wird.

2. Apparat nach Anspruch 1, bei dem das Messsignal eine dem die Lampe (LF) durchfließenden Strom proportionale Messspannung (URm) ist, die durch einen Strom-Spannungs-Wandler bereitgestellt wird, wobei der besagte Strom-Spannungs-Wandler vorzugsweise ein in Serie in der Hauptschleife (CE-SW-LF) geschalteter Widerstand (Rm) ist.

3. Apparat nach Anspruch 2, bei dem eine Freilaufdiode (D) in eine zusätzliche Schleife (Rm-LF-D) einbezogen ist, die - gemeinsam mit der Hauptschleife - die Blitzlichtlampe (LF) und den Strom-Spannungs-Wandler (Rm) aufweist.

4. Apparat nach einem der Ansprüche 1 bis 3, bei dem die Hauptschleife (CE-SW-LF) des Weiteren eine Induktivität (INDUC) aufweist.

5. Apparat nach einem der vorhergehenden Ansprüche, bei dem die Hysterese-Vergleichsvorrichtung (COMP) und der Referenzwert (Uref) derart ausgewählt sind, den die Lampe (LF) durchfließenden Strom an einen Wert zu regulieren, der den Wert desjenigen Stromes, bei dem die Ausdehnung des Lichtbogens die unteren Wände der Lampe (LF) erreicht, im Wesentlichen unterschreitet.

6. Apparat nach einem der vorhergehenden Ansprüche, bei dem der besagte wenigstens eine Kondensator (CE) derart ausgewählt ist, dass das Verhältnis zwischen der Ausgangsspannung an seinen Klemmen (Ui) vor der Entladung und der Endspannung (Uf) am Ende des Strompulses zwischen 2 und 6 und vorzugsweise in der Größenordnung von 4 beträgt.

7. Apparat nach einem der vorhergehenden Ansprüche, bei dem der besagte mindestens eine Kondensator eine Kapazität von größer gleich 13000 Mikrofarad und eine Nennspannung von größer gleich 400 Volt aufweist, wobei der besagte mindestens eine Kondensator vorzugsweise ein Elektrolytkondensator ist.

8. Apparat nach einem der vorhergehenden Ansprüche, bei dem die Kapazität des Kondensators (CE) derart ausgewählt ist, dass der während einer Zeitspanne von 1 Millisekunde bis zum Ende des Strompulses gemessene Mittelwert der Intensität des die Lampe (LF) durchfließenden Stroms zwischen 90 % und 100 % des Mittelwerts der in einer gleichen Zeitspanne am Beginn des Strompulses gemessenen Intensität beträgt.

9. Apparat nach einem der vorhergehenden Ansprüche, bei dem der elektronische Schalter (SW) derart ausgewählt ist, eine Schaltzeit aufzuweisen, die eine Mikrosekunde deutlich unterschreitet, wobei der besagte Schalter vorzugsweise ein IGBT ist.

10. Apparat nach einem der vorhergehenden Ansprüche, bei dem ein elektronisches Steuermodul (COMM) zur Versorgung der Hysterese-Vergleichsvorrichtung (COMP) mit dem Referenzwert (Uref) und zur Steuerung der Freigabe eines Strompulses in die Hauptschleife während einer bestimmten Pulsdauer angepasst ist, wobei die besagte Pulsdauer zwischen 20 ms und 45 ms, vorzugsweise etwa 35 ms beträgt.

11. Apparat nach Anspruch 10, bei dem das Steuermodul (COMM) zur Versorgung des schnellschaltenden Schalters (SW) mit einer Verzögerung (Tinterdit) bestimmter Dauer bei jedem Öffnen dieses Schalters (SW) angepasst ist, während der es ihm untersagt ist, erneut in den geschlossenen Zustand zu kippen, wobei die bestimmte Dauer signifikant länger als die Schaltzeit des Schalters (SW), aber kleiner als die zum Erlöschen der Lampe (LF) notwendige Zeit ist.

12. Apparat nach Anspruch 10 oder 11, bei dem das Steuermodul (COMM) dazu angepasst ist, keine Emission eines neues Strompulses zu steuern, bevor eine bestimmten Zeit, genannt Ruhezeit, seit dem vorhergehenden Strompulses abgelaufen ist, wobei die bestimmte Ruhezeit zwischen einer Sekunde und 10 Sekunden, vorzugsweise etwa 7 Sekunden beträgt.

13. Apparat nach einem der vorhergehenden Ansprüche, bei dem der besagte mindestens eine Kondensator (CE), der elektronische Schalter (SW) und die Hysterese-Vergleichsvorrichtung (COMP) in demselben Gehäuse aufgenommen sind, wobei das besagte Gehäuse ein Volumen von weniger als 5 Litern aufweist.

14. Apparat nach einem der vorhergehenden Ansprüche, bei dem das Gewicht der Gesamtheit des Apparats größer gleich zwei Kilo ist.

15. Verfahren zur Anwendung eines Apparats zur Haarentfernung, der zur Aussendung zumindest eines Lichtpulses angepasst ist, welcher durch einen eine Blitzlichtlampe (LF) durchfließenden und darin einen Lichtbogen bildenden elektrischen Strompuls erzeugt wird, bei dem zumindest ein Kondensator (CE), der zur Freigabe des die Blitzlichtlampe durchfließenden Stroms angepasst ist, ein schnellschaltender elektronischer An/Aus-Schalter (SW) bereitgestellt wird, wobei der elektronische Schalter (SW) und die Blitzlichtlampe (LF) eine Hauptschleife (CE-SW-LF) bilden, **dadurch gekennzeichnet, dass** es im Verlauf der Erzeugung des elektrischen Strompulses die folgenden Schritte umfasst:
- Erzeugung zumindest eines Messsignals (URm), das für den die Lampe (LF) durchfließenden Strom repräsentativ ist,
- Hysterese-Vergleich zwischen dem Messsignal (URm) und einem Referenzwert (Uref);
- Steuern des Schließens des elektronischen Schalters (SW), wenn der Vergleich zwischen dem Messsignal (URm) und dem Referenzwert (Uref) anzeigt, dass der die Lampe (LF) durchfließende Strom kleiner als eine bestimmte Intensität (Icritic) ist; oder
- Steuern des Öffnens des elektronischen Schalters (SW), wenn der Vergleich zwischen dem Messsignal (URm) und dem Referenzwert (Uref) anzeigt, dass der die Lampe (LF) durchfließende Strom größer als eine bestimmte Intensität (Icritic) ist, derart, dass der besagte die Lampe (LF) durchfließende Strom durch hochfrequentes Schalten desselben um die bestimmte Intensität (I critic) kompensiert wird.

16. Verfahren gemäß Anspruch 15, bei dem bei jedem Öffnen des Schalters (SW) ein Verzögerungsschritt (Tinterdit) bestimmter Dauer vorgesehen ist, während der es dem Schalter (SW) untersagt ist, erneut in den geschlossenen Zustand zu kippen, wobei die bestimmte Dauer signifikant länger als die Schaltzeit des Schalters (SW), aber kleiner als die zum Erlöschen der Lampe (LF) notwendige Zeit ist.
